# EUROPÄISCHE PATENTSCHRIFT

(11) **EP 2 074 933 B1**
(45) Veröffentlichungstag und Bekanntmachung des Hinweises auf die Patenterteilung: **02.05.2012**
(21) Anmeldenummer: 08170247.4
(22) Anmeldetag: 28.11.2008
(51) Int. Cl.: A61B 1/04, G06T 7/00, G02B 21/00, A61B 19/00, A61B 5/00, A61B 1/06, G02B 21/16

(54) **Verfahren zur Analyse und Bearbeitung von Fluoreszenzbildern**
Method of analysing and processing fluorescent images
Procédé destiné à l'analyse et au traitement d'images fluorescentes

(30) Priorität: 19.12.2007 CH 19692007
(43) Veröffentlichungstag der Anmeldung: 01.07.2009
(73) Patentinhaber: Kantonsspital Aarau AG, 5001 Aarau (CH)
(72) Erfinder: Hefti, Martin, Dr. med., 5600 Lenzburg (CH); Looser, Herbert, Prof., 8049 Zürich (CH); Landolt, Hans, Prof. Dr. med., 5001 Aarau (CH)
(74) Vertreter: Feldmann, Clarence Paul

(56) Entgegenhaltungen:
- DE-A1- 19 523 806
- DE-U1-202005 021 111
- US-A- 5 408 996
- US-A1- 2004 155 957
- US-A1- 2006 025 692
- ZAAK ET AL: "QUANTIFICATION OF 5-AMINOLEVULINIC ACID INDUCED FLUORESCENCE IMPROVES THE SPECIFICITY OF BLADDER CANCER DETECTION" JOURNAL OF UROLOGY, BALTIMORE, MD, US, Bd. 166, Nr. 5, 1. November 2001 (2001-11-01), Seiten 1665-1669, XP005542895 ISSN: 0022-5347

## Beschreibung

### Technisches Gebiet

Die vorliegende Erfindung beschreibt ein Verfahren zur Analyse und Bearbeitung von Fluoreszenzbildern eines medizinischen Operationsmikroskops.

### Stand der Technik

Schon seit längerer Zeit wird in der Medizin die Fluoreszenz von Tumorgewebe, welche durch die Anreicherung des Tumorgewebes mit speziellen Kontrastmitteln und durch anschliessende Beleuchtung mit UV-Licht erreicht wird, beispielsweise zur Unterstützung von Resektionen von Gewebeteilen im Operationssaal genutzt.

Bei dieser, als intraoperativer Fluoreszenzdetektion bezeichneten Methode, wird einem Patienten beispielsweise mit einem Glioblastom vor der Hirntumoroperation eine natürliche, körpereigene Substanz (5-Aminolävulinsäure oder kurz 5-ALA) verabreicht. 5-ALA, das im Rahmen der Bildung des Blutfarbstoffes (Hämoglobin) im Körper entsteht und für die Blutbildung unerlässlich ist, hat die Eigenschaft in bösartigem Hirntumorgewebe in den Farbstoff Protoporphyrin IX umgewandelt zu werden. Das Protoporphyrin IX häuft sich in neoplastischen Zellen und damit besonders in Tumorgewebe an. Protoporphyrin IX zeigt eine typische Fluoreszenz bei einer Wellenlänge von etwa 635 nm, wenn es mit UV-Licht bestrahlt wird. Die Protoporphyrin IX Moleküle absorbieren das anregende UV-Licht und strahlen ein langwelligeres energieärmeres Fluoreszenzlicht ab, so dass das Tumorgewebe rot leuchtet.

Da der zu entfernende Tumor häufig einen breiten Zellsaum aufweist, welcher das Normalgewebe infiltriert, oder Tumorzellansammlungen von Normalgewebe umgeben sind, ist es schwierig, nur die Tumorzellen bei minimaler Schädigung des Normalgewebes zu entfernen. Der Operateur sieht zwar im Fluoreszenzbild eine RotFluoreszenz, muss aber eigenständig beurteilen, wie viel Gewebe wegzunehmen ist.

Vor allem im Bereich der Gehirnchirurgie, beispielsweise bei der Resektion von Gliomen ist es vom Operateur gewünscht, das Tumorgewebe möglichst vollständig zu resezieren, und gleichzeitig möglichst wenig bis kein Normalgewebe zu beschädigen, damit zusätzliche neurologische Störungen vermieden werden können. Mit den bislang bekannten Methoden ist eine optimierte Resektion, welche auf einer objektiven Bestimmung der Tumorgrenzen basiert, nicht möglich.

Es sind Operationsmikroskope bekannt, welche als Hilfsmittel bei der Operation eingesetzt werden und dem Operateur eine vergrösserte Abbildung des interessierenden Körperbereiches des Patienten zeigen und weitere unterstützende Merkmale aufweisen.

In der DE 202005021111U1 ist ein kombiniertes diagnose- und therapieuntersützendes System offenbart. Das beschriebene System umfasst mindestens eine Lichtquelle, eine Bilddetektionseinrichtung, ein Bildverarbeitungssystem und ein Projektionssystem. Bildinformationen werden von der Bilddetektionseinrichtung detektiert und im Bildverarbeitungssystem weiterverarbeitet. Aus der Bildverarbeitung resultieren Zusatzinformationen, welche dem Operateur derart zugänglich gemacht werden, dass diese Zusatzinformationen mit Hilfe des Projektionssystems in den Operationsbereich hinein projiziert werden. Die Projektion kann mittels eines Beamers in den Operationsbereich, oder beispielsweise in das Okular oder den Monitor des Operationsmikroskopes erfolgen.

Der Operateur sieht durch die Projektion das Realbild des Operationsfeldes, welchem ein generiertes Bild überlagert ist, in welchem beispielsweise Normalgewebe grün eingefärbt erscheint, oder erkranktes Gewebe durch eine höhere Intensität markiert ist. Diese Zusatzinformationen werden im Bildverarbeitungssystem offline generiert.

Das oben erwähnte Dokument beschreibt zwar die photodynamische Diagnose von Tumorgewebe anhand der Anregung von Fluoreszenzstrahlung, es wird aber nicht offenbart, wie und ob das Bildverarbeitungssystem die zu entfernenden Gewebeteile analysieren kann, oder die Schnittlinien bestimmen und darstellen kann, damit der Operateur die Resektion durch Zusatzinformationen unterstützt durchführen kann.

Der Schwerpunkt der Offenbarung der DE 202005021111U1 liegt auf der Projektion von Zusatzinformationen in den Operationsbereich, wobei nicht erwähnt wird, wie die Auswertung und die Bestimmung und Berechnung der Zusatzinformation stattfindet. Gemäss des oben erwähnten Schutzrechts kann durch einen Fachmann nicht nachvollzogen werden, wie Normalgewebe von Tumorgewebe unterschieden wird, weshalb es dem Operateur weiterhin selbst überlassen wird, die Intensität der Fluoreszenz bzw. das projizierte Bild zu beurteilen und das zu entfernende tumorale Gewebe zu bestimmen. Da die optische Wahrnehmung und Beurteilung der Fluoreszenz stark vom jeweiligen Operateur abhängt, variiert das Ausmass der Resektion zum Teil beträchtlich.

Um die Zeit bis zum Wiederauftreten von Rezidivtumoren zu verlängern ist es unbedingt erforderlich möglichst alle Tumorzellen zu entfernen.

Da die Farbwahrnehmung von Operateur zu Operateur unterschiedlich ist und das subjektive Befinden eines Fluoreszenzbildes von der Umgebung und Beleuchtung im Operationssaal abhängt, ist bisher keine objektive Bestimmung von Tumorgrenzen, welche vom Operateur unabhängig ist, offenbart.

Ein aus der Druckschrift US-A-5 408 996 bekanntes Verfahren bestimmt die Rotfluoreszenz nach Grünanregung mittels gefilterter CCD-Kamera und stellt anschliessend die Intensitätswerte unter und oberhalb eines fest vorgegebenen Schwellwerts in unterschiedlichen Farben dar.

### Darstellung der Erfindung

Die vorliegende Erfindung hat sich zur Aufgabe gestellt ein Verfahren zu schaffen, welches eine quantifizierbare objektive und reproduzierbare Bestimmung der Grenzen von Tumorgewebe erlaubt, sodass die subjektive Wahrnehmung sowie die Erfahrung des Operateurs keine Rolle spielt.

Diese Aufgabe und die damit verbundene Unterstützung der Resektion von Tumorgewebe, wodurch nur ein minimaler Teil des an den Tumor angrenzenden Normalgewebes entfernt wird und damit zusätzliche neurologische Störungen weitgehend vermieden werden können, löst das erfindungsgemässe Verfahren.

Eine weitere Aufgabe des Erfindungsgegenstandes liegt in der Verbesserung der Lebensqualität und der Steigerung der Lebenserwartung der Patienten durch die erreichbare nahezu vollständige Tumorentfernung.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1a: zeigt eine schematische Darstellung eines Fluoreszenzmikroskops, welches als Operationsmikroskop eingesetzt wird, während
- Figur 1b: ein Fluoreszenzspektrum zeigt, wobei die Intensität der Strahlung gegen die Wellenlänge aufgetragen ist und die Fluoreszenzanregung sichtbar ist.
- Figur 2: zeigt ein Fluoreszenzbild eines beleuchteten und reflektierenden Operationsbereiches, während
- Figur 3: ein Fluoreszenzbild des beleuchteten Operationsbereiches der Figur 2 mit überlagerten kritischen Grenzintensitätslinien zeigt.
- Figur 4: zeigt ein Fluoreszenzbild entsprechend Figur 3, wobei Bereiche weiterer Intensitäten durch zusätzlich Konturlinien abgegrenzt sind, während
- Figur 5: einen reinen Konturplot der Grenzintensitätslinie (durchgehend schwarz) und der zusätzlichen Konturlinien darstellt, welcher mit dem Bildverarbeitungssystem erzeugt wurde.
- Figur 6: zeigt ein Falschfarbenbild des Operationsbereiches mit unterschiedlichen Konturlinien und unterschiedlich eingefärbten Bereichen.

### Beschreibung

Nachdem einem Gliom-Patienten eine Substanz zur Generierung eines Tumorerkennungsmerkmales, bevorzugt 5-Aminolävulinsäure zur Generierung einer Protoporphyrin-fluoreszenz oral verabreicht wurde, der Patient für die Operation vorbereitet, anästhesiert und ein zu operierender Operationsbereich 20 zugänglich gemacht worden ist, findet die Resektion von Tumorgewebe 22 unter Verwendung eines Operationsmikroskops statt.

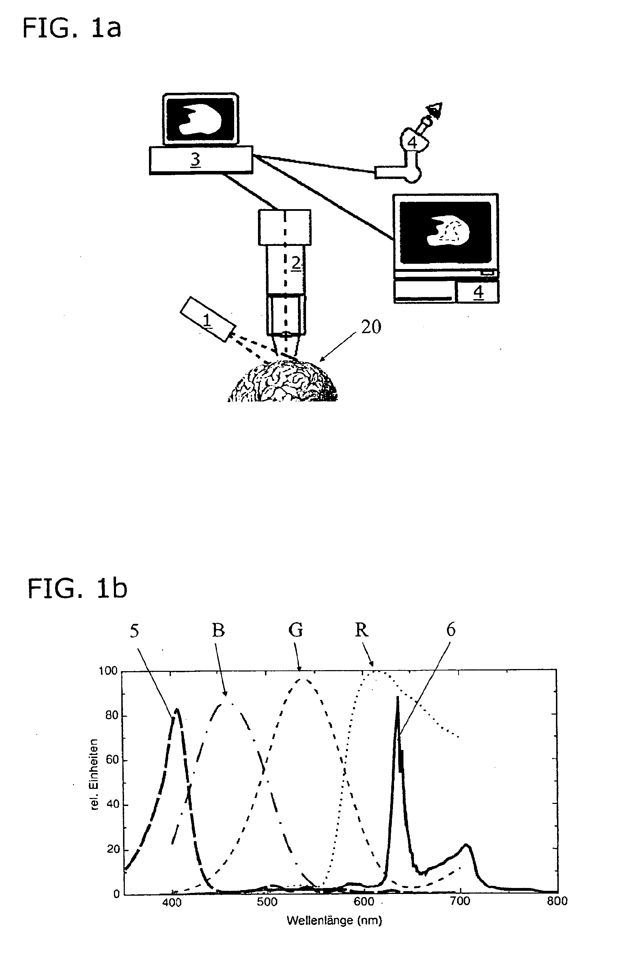

Eine mögliche Anordnung eines Operationsmikroskops ist schematisch in Figur 1a gezeigt. Der Operationsbereich 20 wird von einer Anregungseinrichtung 1 mit UV-Strahlung ausgeleuchtet, welche im Wellenlängenbereich von etwa 400 nm und damit im blauen sichtbaren Wellenlängenbereich liegt. Die fluoreszenzanregende UV-Strahlung kann beispielsweise von einer mit Filtern versehenen Xenonlichtquelle oder von einem Laser stammen. Die 5-Aminolävulinsäure synthetisiert zu Protoporphyrin IX, welches fluoreszierende Eigenschaften besitzt und welches sich selektiv in pathologisch veränderten Zellen anhäuft.

Das Fluoreszenzspektrum der Figur 1b zeigt neben einer Anregungslinie 5 der fluoreszenzanregenden UV-Strahlung der Anregungseinrichtung 1 bei etwa 400nm, eine Emissionslinie 6 der abgestrahlten Strahlung des fluoreszierenden Protoporphyrin IX im sichtbaren Spektralbereich von etwa 600 nm bis 700 nm. Das Tumorgewebe 22 fluoresziert und strahlt rotes Licht gemäss der Emissionslinie 6 im sichtbaren Spektralbereich ab, wobei die Intensität des emittierten Lichts mit der Konzentration von intrazellurärem Protoporphyrin IX korreliert ist.

Eine Bilddetektionseinrichtung 2 sammelt und bündelt das emittierte Licht mittels optischer Komponenten auf einem Detektor, welcher digitale Bildinformationen in Form eines Fluoreszenzbildes 10, welches vom Operationsbereich 20 ausgesandt wird, erzeugt. Die eigentliche Detektion in der Bilddetektionseinrichtung 2 kann von einer digitalen CCD-Kamera oder von einem Fluoreszenzspektrometer durchgeführt werden.

Durch die Detektion der emittierenden Strahlung mit einer CCD-Kamera, welche die Intensitäten des Rotanteils R, des Grünanteils G und des Blauanteils B einzeln detektiert, sind im Falle der Emissionslinie 6 im Bereich oberhalb von 600 nm keine zusätzlichen Filter mehr notwendig, um optimale Messergebnisse zu erhalten. Der grosse Abstand zwischen der Anregungslinie 5 und der Emissionslinie 6 ist also bei Verwendung einer CCD-Kamera vorteilhaft, da die Emissionslinie 6 nur innerhalb des Rotanteils R liegt und damit die Detektion nicht von der blauen Anregungslinie 5 im Blauanteil B gestört wird. Desweiteren ist die Quanteneffektivität der meissten CCD-Sensoren im Bereich des roten Lichtes am grössten, womit also die höchste Sensitvität einer CCD-Kamera im Rotbereich liegt.

Die Bilddetektionseinrichtung 2 nimmt für jeden Bildpunkt verschiedene Helligkeitswerte des Rotanteils R, des Grünanteils G und des Blauanteils B auf. Wird ein 8 Bit Sensor gewählt, können 256 verschiedene Helligkeitswerte aufgenommen werden. Wird ein 16 Bit Sensor gewählt, können 65536 verschiedene Helligkeitswerte aufgenommen werden und als digitales Fluoreszenzbild 10 direkt an eine Darstellungseinrichtung 4 weitergeleitet und dort dargestellt werden. Die Darstellungseinrichtung 4 kann ein Okular oder Binokular, ein Monitor oder ähnliches sein. Der Operateur beobachtet den gesamten Eingriff mit Hilfe der Darstellungseinrichtung 4.

Zusätzlich wird das digitale Fluoreszenzbild 10 auch an das Bildverarbeitunngssystem 3 weitergeleitet. Das Bildverarbeitungssystem 3 umfasst eine Rechnereinheit mit mindestens einem Schreib-/Lesespeicher und einem Computerprogramm, welches die Auswertung und die Verarbeitung der Fluoreszenzbilder ausführt und die Ausgabe von generierten Linienprofilbildern 11 und generierten Falschfarbenbildern 12 und die Überlagerung des Fluoreszenzbildes 10 mit den generierten Bildern bewerkstelligt.

Da die Fluoreszenz im roten Spektralbereich liegt und das mit Protoporphyrin IX angereicherte Tumorgewebe 22 rot leuchtet, erfolgt eine Auswertung und Verarbeitung des Rotanteils R des Fluoreszenzbildes 10, um eine quantitative Bestimmung der Ausmasse und der Grenzen des Tumorgewebes 22 zu erreichen. Der erste Auswertungsschritt des Bildverarbeitungssystems 3 ist eine Extraktion des Rot-Kanals aus dem aufgenommenen Fluoreszenzbild 10.

Das Fluoreszenzbild 10 weist verschiedene Bereiche mit unterschiedlich hoher Lichtintensität im roten Spektralbereich auf. Wahlweise bestimmt das Bildverarbeitungssystem 3 oder der Operateur den Bereich im Fluoreszenzbild 10 mit einer Maximalintensität 26.
Das Bildverarbeitungssystem 3 ist in der Lage die Maximalintensität 26 durch Vergleich der Intensitäten aller Pixel des Rotanteils des Fluoreszenzbildes 10 zu ermitteln und als Maximalintensität zu speichern. Für die manuelle Bestimmung der Maximalintensität 26 ist eine Eingabevorrichtung, beispielsweise eine Computermaus, mit dem Bildverarbeitungssystem 3 verbunden, mit welcher der Operateur den seiner Meinung nach hellsten Bereich von Pixeln im Fluoreszenzbild 10 wählt. Während eine automatische Bestimmung der Maximalintensität 26 reproduzierbar immer die tatsächlich höchsten Intensitätswerte als Maximalintensität 26 bestimmt, verhindert die manuelle Bestimmung der Maximalintensität 26 durch den Operateur, dass mögliche Bildfehler im Fluoreszenzbild 10 durch die automatische Bestimmung als Maximalintensität 26 interpretiert werden. Damit eine Farbenfehlsichtigkeit des Operateurs nicht zu Problemen bei der manuellen Bestimmung der Maximalintensität 26 führt, ist es vorteilhaft, dass der interessierende Rotkanal des Fluoreszenzbildes 10 mit bekannten Mitteln in ein Graustufenbild umgewandelt wird, bevor die Maximalintensität 26 im hellsten Bereich bestimmt wird.

In einem nächsten Schritt wird ein Schwellwert der Intensität vom Bildverarbeitungssystem 3 definiert, welcher einen Bruchteil der Maximalintensität 26 darstellt. Der Schwellwert stellt die Intensität der detektierten Fluoreszenz-Strahlung dar, welche zwischen der Intensität von Normalgewebe 21 und Tumorgewebe 22 liegt. Wie Versuche gezeigt haben, charakterisiert ein Schwellwert im Bereich von 30% der Maximalintensität 26 die Schwelle zwischen gesundem Normalgewebe 21 und Tumorgewebe 22 ziemlich genau.

Flächen mit Pixeln im Fluoreszenzbild 10, welche eine Intensität oberhalb dieses Schwellwertes aufweisen, kennzeichnen Tumorgewebe 22, während Gewebe, welches Licht mit Intensitäten unterhalb des Schwellwertes abstrahlt und als Fläche im Fluoreszenzbild 10 sichtbar ist, als Normalgewebe 21 eingestuft wird. Der gewünschte Schwellwert kann im Bildverarbeitungssystem 3 hinterlegt werden und kann falls nötig auch verändert werden. Klinische Versuche und Auswertungen bereits durchgeführter Operationen im Hinblick auf das Wiederauftreten von Tumorgewebe 22 haben einen Schwellwert von 33% der Maximalintensität 26 als besonders vorteilhaft bestätigt.

Nach Bestimmung der Maximalintensität 26 und des Schwellwertes, wird ein binäres Bild erstellt, wobei Pixel des R-Kanals des Fluoreszenzbildes 10 mit Intensitäten unterhalb des Schwellwertes und Pixel mit Intensitäten oberhalb des Schwellwertes unterschieden werden. Die Grenzen zwischen den Bereichen mit Intensitäten grösser als der Schwellwert und Bereichen mit Intensitäten kleiner als der Schwellwert werden durch übliche Verfahren der digitalen Bildverarbeitung bestimmt und hervorgehoben. Durch morphologische Basisoperationen der Bildbearbeitung, beispielsweise durch Dilatation und/oder Erosion mit einem strukturierenden Element, zum Beispiel in Form einer 3x3 Matrix aus Einsen, werden die Grenzen zwischen verschiedenen Bildbereichen bestimmt. In der Praxis sind Kombinationen und Mehrfachanwendungen der Dilatation und Erosion sinnvoll, beispielsweise Opening und Closing, wodurch Kanten detektiert werden. Durch Anwendung dieser bekannten Verfahren der digitalen Bildbearbeitung werden Bildinformationen generiert, welche die Grenze zwischen Tumorgewebe 22 und Normalgewebe 21 als mehrere Pixel breite Grenzintensitätslinie 25 beinhalten.

Die Breite der Grenzintensitätslinie 25 ist variierbar und wird unter anderem durch das strukturierende Element bestimmt, welches Bestandteil der morphologischen Operatoren ist. Die Breite der Grenzintensitätslinien 25 kann variiert werden, wenn das strukturierende Element entsprechend anders im Bildverarbeitungssystem 3 definiert wird. Es sind auch weitere Operationen, wie Tief- bzw. Hochpassfilterung oder der Gradient-Operator anwendbar, um die Grenzintensitätslinien 25 zu erzeugen.

Die Grenzintensitätslinie 25 ist in sich geschlossen und umschliesst einen Tumorgewebebereiche 27 in dem sich Pixel mit einer höheren Intensität als der definierte Schwellwert befinden. Die Pixel in den Tumorgewebebereichen 27 mit Intensitäten grösser als der Schwellwert, charakterisieren Tumorgewebe 22. Ausserhalb der umschlossenen Grenzintensitätslinie 25 weisen die Pixel Intensitäten auf, welche unterhalb des Schwellwertes liegen, womit das Gewebe im Bereich dieser Pixel als Normalgewebe 21 definiert ist und der Bereich Normalgewebebereich 23 genannt wird.

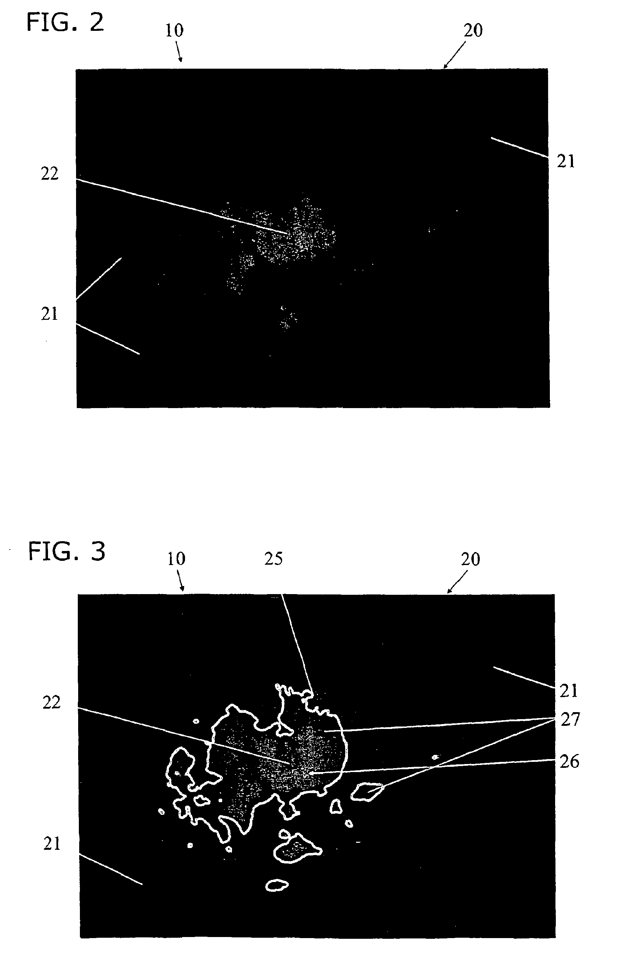

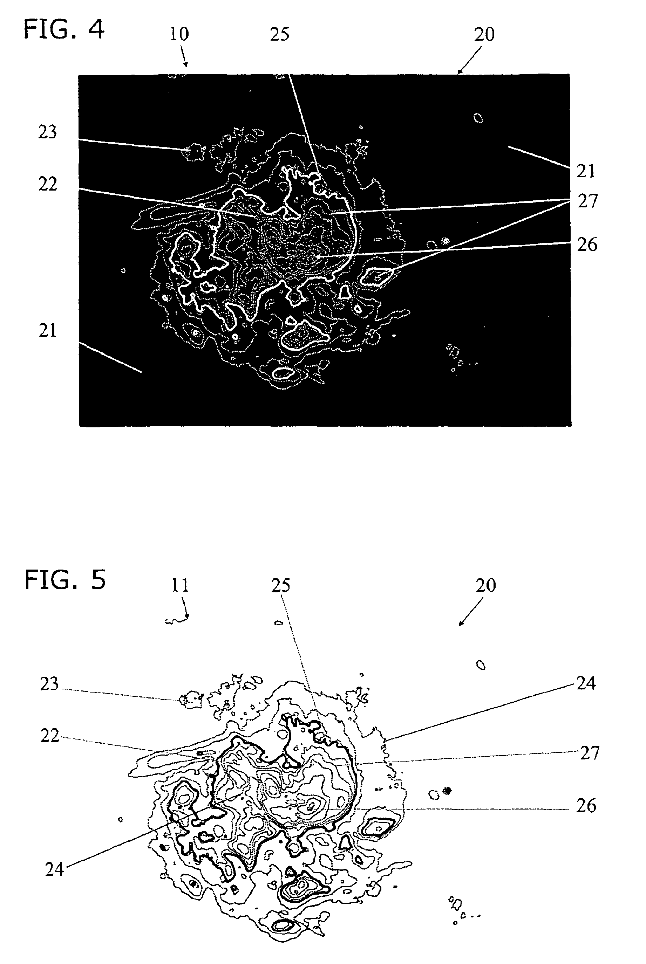

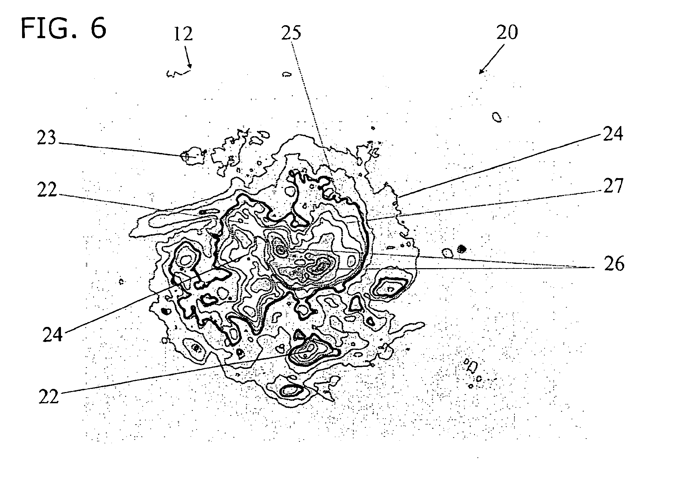

Das Bildverarbeitungssystem 3 erstellt aus den Grenzintensitätslinien 25 ein Linienprofilbild 11 welches dem aufgenommenen Fluoreszenzbild 10 überlagert werden kann und mittels der Darstellungseinrichtung 4 darstellbar ist. Figur 3 zeigt ein Fluoreszenzbild 10, welchem ein Linienprofilbild 11 gemäss Figur 5, umfassend mehrere Bereiche, welche von jeweils einer geschlossenen Grenzintensitätslinie 25 umsäumt werden, überlagert wird. Die erzeugten Linienprofilbilder 11 können wahlweise auch für sich, wie in Figur 5 gezeigt, ohne Überlagerung auf das Fluoreszenzbild 10, oder als Falschfarbenbild 12, wie in Figur 6 gezeigt dargestellt und vom Operateur ausgewertet und zur Unterstützung der Operation gewählt werden.

Während das digitale Fluoreszenzbild 10 direkt ohne Bearbeitung in Echtzeit durch die Darstellungseinrichtung 4 angezeigt wird, können vom Bildverarbeitungssystem 3 erzeugte Linienprofilbilder 11 und Falschfarbenbilder 12 dem Fluoreszenzbild 10 überlagert und angezeigt werden. Auch diese Überlagerung erfolgt in Echtzeit. Damit kann der Operateur in seiner gewohnten Darstellungseinrichtung 4 verarbeitete und berechnete Zusatzinformationen des Operationsbereiches 20 während der Resektion des Tumorgewebes 22 einsehen.

Um Operationen zu einem späteren Zeitpunkt auszuwerten und die Schwellwertbestimmung durch Studien zu optimieren, ist es vorgesehen die aufgenommenen Fluoreszenzbilder 10 und die generierten Linienprofilbilder 11 und Falschfarbenbilder 12 für Studienzwecke im Bildverarbeitungssystem 3 auf einer Festplatte oder einem anderen Festspeicher zu speichern.

Entsprechend dem oben beschriebenen Verfahren kann es gewünscht sein, dass neben den dargestellten Grenzintensitätslinien 25 zusätzliche Konturlinien 24 dargestellt werden.

Die Konturlinien 24 umgeben Bereiche im Fluoreszenzbild 10 deren Intensitäten einen festen definierten Abstand zum definierten Schwellwert haben. Dabei können beispielsweise Intensitätsunterschiede von 10% zwischen Schwellwert und Maximalintensität 26 gewählt werden. Die Konturlinien 24 sind im Linienprofilbild 11 der Figur 5 gezeigt. Um die verschiedenen Konturlinien 24 zu unterscheiden, können die Konturlinien 24 durch die Darstellungseinrichtung 4 unterschiedlich, beispielsweise strichliniert oder punktiert dargestellt werden. Jede Konturlinie 24 umschliesst eine Fläche von Pixeln, deren Intensität in einem bestimmten Verhältnis zum Schwellwert stehen. Dabei kann es gewünscht sein, dass die Konturlinien 24 Normalgewebebereiche 23 und/oder Tumorgewebebereiche 27 mit Intensitäten oberhalb des Schwellwertes bis zur Maximalintensität umschliessen.

Die von den Konturlinien 24 umschlossenen Bereiche, welche innerhalb eines von einer Grenzintensitätslinie 25 umschlossenen Tumorgewebebereiches 27 liegen, charakterisieren die verschieden stark fluoreszierenden Bereiche innerhalb des Tumorgewebebereiches 27. Es kann auch gewünscht sein, Normalgewebebereiche 23 ausserhalb eines durch eine Grenzintensitätslinie 25 umschlossenen Tumorgewebebereiches 22 durch eine Konturlinie 24 begrenzt darzustellen. Dies ist ebenfalls in Figur 5 dargestellt.

Auch im Falschfarbenbild (oder auch farbcodiertem Bild) 12 der Figur 6 sind diese zusätzlichen Konturlinien 24 eingezeichnet, wobei zur Verdeutlichung der unterschiedlich intensiv strahlenden Bereiche des Fluoreszenzbildes 10, die von den Konturlinien 24 umgebenden Tumorgewebebereiche 27 unterschiedlich eingefärbt sind. Die Bestimmung der Grenzintensitätslinien 25, der Konturlinien 24, sowie die Einfärbung der unterschiedlich fluoreszierenden Bereiche, führt das Bildverarbeitungssystem 3 aus. Der Operateur kann sich wahlweise das Fluoreszenzbild 10 mit überlagertem Linienprofilbild 11 oder Falschfarbenbild 12, oder das Linienprofilbild 11 oder das Falschfarbenbild 12 bei ausgeblendetem Fluoreszenzbild 10 anzeigen lassen.

Um individuelle Fehler bei der Bestimmung des Ausmasses von Tumorgewebe 22 zu verringern, wurde das beschriebene Verfahren zur quantifizierten, objektiven und reproduzierbaren Bestimmung der Grenzen von Tumorgewebe 22 entwickelt. Die Bestimmung des Schwellwertes kann dabei entweder durch den Operateur stattfinden, oder durch das Bildverarbeitungssystem 3 definiert werden.

Während der Operation werden kontinuierlich Fluoreszenzbilder 10 aufgenommen und die verschiedenen Bereiche anhand der zu Anfang bestimmten Maximalintensität 26 und des Schwellwertes analysiert und die Grenzintensitätslinien 25 und die Konturlinien 24 ständig neu berechnet und dargestellt. So werden die Tumorgewebebereiche 27, welche von Grenzintensitätslinien 25 umsäumt werden im Verlauf der Operation flächenmässig verkleinert, indem das Tumorgewebe 22 vollständig reseziert wird. Sobald keine Strahlung mit Intensitäten oberhalb des Schwellwertes detektiert wird, ist das gesamte als Tumorgewebe klassifizierte Gewebe entfernt und die Operation beendet.

### Bezugszeichenliste

- 1: Anregungseinrichtung
- 2: Bilddetektionseinrichtung
- 3: Bildverarbeitungssystem
- 4: Darstellungseinrichtung

- 5: Anregungslinie
- 6: Emissionslinie

- 10: Fluoreszenzbild
- 11: Linienprofilbild
- 12: Falschfarbenbild

- 20: Operationsbereich
- 21: Normalgewebe
- 22: Tumorgewebe
- 23: Normalgewebebereich
- 24: Konturlinie (sämtliche)
- 25: Grenzintensitätslinie (speziell)
- 26: Maximalintensität (entspricht 100% Intensität)
- 27: Tumorgewebebereich

## Patentansprüche

1. Verfahren zur Analyse und Bearbeitung von Fluoreszenzbildern eines medizinischen Operationsmikroskops, **dadurch gekennzeichnet, dass**
- ein Operationsbereich (20) mit UV-Strahlung einer Anregungseinrichtung (1) bestrahlt wird,
- eine Bilddetektionseinrichtung (2) ein digitales Fluoreszenzbild (10) aufnimmt und an ein Bildverarbeitungssystem (3) weiterleitet in welcher
- der Rotkanal (R) aus dem Fluoreszenzbild (10) extrahiert wird, und anschliessend
- eine Maximalintensität (26) festgelegt wird, wonach
- ein Schwellwert als ein prozentualer Anteil der Maximalintensität (26) ermittelt wird,
- Grenzintensitätslinien (25) bestimmt werden, welche Tumorgewebebereiche (27) umschliessen, deren Pixel Intensitäten oberhalb des definierten Schwellwertes aufweisen, woran anschliessend
- ein Linienprofilbild (11) berechnet wird, welches die Grenzintensitätslinien (25) von Tumorgewebebereichen (27) umfassen und
- dem Fluoreszenzbild (10) in einer Darstellungseinrichtung (4) überlagert dargestellt wird, wobei die nicht von Grenzintensitätslinien (25) umschlossenen Bereiche als Normalgewebebereiche (23) definiert werden.

2. Verfahren zur Analyse und Bearbeitung von Fluoreszenzbildern nach Anspruch 1, **dadurch gekennzeichnet, dass** der Schwellwert mindestens annähernd 30% der Maximalintensität (26) beträgt.

3. Verfahren zur Analyse und Bearbeitung von Fluoreszenzbildern nach Anspruch 2, **dadurch gekennzeichnet, dass** der Schwellwert 33% der Maximalintensität (26) beträgt.

4. Verfahren zur Analyse und Bearbeitung von Fluoreszenzbildern nach Anspruch 1, **dadurch gekennzeichnet, dass** die Maximalintensität (26) selbstständig automatisch durch das Bildverarbeitungssystem (3) bestimmbar ist.

5. Verfahren zur Analyse und Bearbeitung von Fluoreszenzbildern nach Anspruch 1, **dadurch gekennzeichnet, dass** die Maximalintensität (26) manuell durch einen Operateur mittels einer, mit dem Bildverarbeitungssystem (3) verbundenen Eingabevorrichtung bestimmbar ist, wobei die hellsten Pixel des Fluoreszenzbildes (10) manuell ausgewählt werden.

6. Verfahren zur Analyse und Bearbeitung von Fluoreszenzbildern nach Anspruch 1, **dadurch gekennzeichnet, dass** der Rotkanal (R) des Fluoreszenzbildes (10) vor der Bestimmung der Grenzintensitätslinien (25) vom Bildverarbeitungssystem (3) in ein binäres Bild umgewandelt wird.

7. Verfahren zur Analyse und Bearbeitung von Fluoreszenzbildern nach Anspruch 6, **dadurch gekennzeichnet, dass** die Grenzintensitätslinien (25) mittels morphologischer Basisoperationen der Bildbearbeitung, beispielsweise durch Dilatation und/oder Erosion mit einem strukturierenden Element, ermittelt werden, welche die Grenzen zwischen Tumorgewebe 22 und Normalgewebe 21 darstellt.

8. Verfahren zur Analyse und Bearbeitung von Fluoreszenzbildern nach Anspruch 7, **dadurch gekennzeichnet, dass** das strukturierende Element der Dilatation und/oder Erosion eine 3x3 Matrix aus Einsen ist.

9. Verfahren zur Analyse und Bearbeitung von Fluoreszenzbildern nach Anspruch 8, **dadurch gekennzeichnet, dass** die Breite der Grenzintensitätslinien (25) variierbar ist.

10. Verfahren zur Analyse und Bearbeitung von Fluoreszenzbildern nach Anspruch 6, **dadurch gekennzeichnet, dass** die Grenzintensitätslinien (25) mittels Tief- bzw. Hochpassfilterung oder die Anwendung des Gradient-Operators generiert werden.

11. Verfahren zur Analyse und Bearbeitung von Fluoreszenzbildern nach Anspruch 1, **dadurch gekennzeichnet, dass** das Bildverarbeitungssystem (3) geschlossene Konturlinien (24) aus dem Rotkanal (R) des Fluoreszenzbildes (10) generiert, welche einen festen definierten Abstand zum definierten Schwellwert aufweisen und die Grenzen zwischen Flächen mit verschiedenen Intensitäten oberhalb und unterhalb des Schwellwertes darstellen.

12. Verfahren zur Analyse und Bearbeitung von Fluoreszenzbildern nach Anspruch 1, **dadurch gekennzeichnet, dass** ein Falschfarbenbild (12) von der Darstellungseinrichtung (4) angezeigt wird, welches die Normalgewebebereiche (23) und die Tumorgewebebereiche (27) unterschiedlich gefärbt darstellt.

13. Verfahren zur Analyse und Bearbeitung von Fluoreszenzbildern nach Anspruch 12, **dadurch gekennzeichnet, dass** die Überlagerung des Fluoreszenzbildes (10) mit dem Linienprofilbild (11) und/oder mit dem Falschfarbenbild (12) ein- und ausschaltbar ist.

14. Computerprogrammprodukt zum Bearbeiten von Fluoreszenzbildern eines medizinischen Operationsmikroskops nach mindestens einem der Ansprüche 1 bis 13, welches Programmteile zur Durchführung eines Verfahrens aufweist, wobei das Computerprogrammprodukt folgende Schritte durchführt:
a) Extraktion des Rotkanals (R) des Fluoreszenzbildes
b) Ermittlung der Maximalintensität (26)
c) Ermittlung eines definierten Schwellwertes der Intensität als prozentualer Anteil der Maximalintensität (26)
d) Generierung von Grenzintensitätslinien (25) die Tumorgewebebereiche (27) umschliessen, deren Pixel Intensitäten oberhalb des definierten Schwellwertes aufweisen, mittels morphologischer Basisoperationen der Bildbearbeitung, beispielsweise durch Dilatation und/oder Erosion mit einem strukturierenden Element
e) Generierung eines Linienprofilbildes (11) welches die generierten Grenzintensitätslinien (25) umfaßt und
f) Überlagerung des aufgenommenen Fluoreszenzbildes (10) mit dem Linienprofilbild (11) wobei die nicht von Grenzintensitätslinien (25) umschlossenen Bereiche als Normalgewebebereiche (23) definiert werden.

## Claims

1. A method for analysing and processing fluorescent images of a medical surgical microscope, **characterised in that**
- a surgical region (20) is irradiated with UV radiation from an excitation device (1),
- an image detection device (2) records a digital fluorescent image (10) and forwards it to an image processing system (3), in which
- the red channel (R) is extracted from the fluorescent image (10), and then
- a maximum intensity (26) is determined, and then
- a threshold value is established as a percentage amount of the maximum intensity (26),
- limit intensity lines (25) which surround tumour tissue regions (27) of which the pixels have intensities above the defined threshold value are determined, and then
- a line profile image (11) is calculated which includes the limit intensity lines (25) of tumour tissue regions (27) and
- is superimposed on the fluorescent image (10) in a display device (4), the regions not surrounded by limit intensity lines (25) being defined as normal tissue regions (23).

2. The method for analysing and processing fluorescent images according to claim 1, **characterised in that** the threshold value is at least approximately 30 % of the maximum intensity (26).

3. The method for analysing and processing fluorescent images according to claim 2, **characterised in that** the threshold value is 33 % of the maximum intensity (26).

4. The method for analysing and processing fluorescent images according to claim 1, **characterised in that** the maximum intensity (26) can be determined independently and automatically by the image processing system (3).

5. The method for analysing and processing fluorescent images according to claim 1, **characterised in that** the maximum intensity (26) can be determined manually by a surgeon using an input device connected to the image processing system (3), the brightest pixels of the fluorescent image (10) being selected manually.

6. The method for analysing and processing fluorescent images according to claim 1, **characterised in that** the red channel (R) of the fluorescent image (10) is converted into a binary image before the limit intensity lines (25) are determined by the image processing system (3).

7. The method for analysing and processing fluorescent images according to claim 6, **characterised in that** the limit intensity lines (25) are established by means of basic morphological operations of image processing, for example by dilatation and/or erosion with a structuring element, and represent the borders between tumour tissue (22) and normal tissue (21).

8. The method for analysing and processing fluorescent images according to claim 7, **characterised in that** the structuring element of dilatation and/or erosion is a 3x3 matrix of ones.

9. The method for analysing and processing fluorescent images according to claim 8, **characterised in that** the width of the limit intensity lines (25) is variable.

10. The method for analysing and processing fluorescent images according to claim 6, **characterised in that** the limit intensity lines (25) are generated by low-pass filtering and/or high-pass filtering or by using the gradient operator.

11. The method for analysing and processing fluorescent images according to claim 1, **characterised in that** the image processing system (3) generates closed contour lines (24) from the red channel (R) of the fluorescent image (10) which have a fixed defined distance from the defined threshold value and represent the borders between areas of different intensities above and below the threshold value.

12. The method for analysing and processing fluorescent images according to claim 1, **characterised in that** a false-colour image (12) is displayed by the display device (4) and shows the normal tissue regions (23) and the tumour tissue regions (27) in different colours.

13. The method for analysing and processing fluorescent images according to claim 12, **characterised in that** the superimposition of the line profile image (11) and/or of the false-colour image (12) on the fluorescent image (10) can be activated and deactivated.

14. A computer program product for processing fluorescent images of a medical surgical microscope according to at least one of claims 1 to 13, said computer program product comprising program parts to carry out a method and carrying out the following steps:
a) extracting the red channel (R) of the fluorescent image,
b) establishing the maximum intensity (26),
c) establishing a defined threshold value of the intensity as a percentage amount of the maximum intensity (26),
d) generating limit intensity lines (25) which surround tumour tissue regions (27) of which the pixels have intensities above the defined threshold value by means of basic morphological operations of image processing, for example by dilatation and/or erosion with a structuring element,
e) generating a line profile image (11) which includes the generated limit intensity lines (25), and
f) superimposing the line profile image (11) on the fluorescent image (10) thus recorded, the regions not surrounded by limit intensity lines (25) being defined as normal tissue regions (23).

## Revendications

1. Procédé d'analyse et de traitement des images fluorescentes d'un microscope opératoire médical, **caractérisé en ce que** :
- une zone d'opération (20) est irradiée par un rayonnement UV d'un dispositif d'excitation (1),
- un dispositif de détection d'image (2) enregistre une image fluorescente numérique (10) et la transmet à un système de traitement d'image (3), dans lequel
- la composante rouge (R) est extraite de l'image fluorescente (10), et puis,
- une intensité maximale (26) est fixée, et ensuite
- une valeur seuil est déterminée en pourcentage de l'intensité maximale (26),
- des lignes d'intensité limite (25) sont déterminées qui entourent des zones de tissu tumoral (27) dont les pixels présentent des intensités au-dessus de la valeur seuil définie, et par la suite
- une image de profil linéaire (11) est calculée qui comprend les lignes d'intensité limite (25) de zones de tissu tumoral (27), et
- qui est visualisée sur un dispositif de visualisation (4) en superposition avec l'image fluorescente (10), les zones non entourées par des lignes d'intensité limite (25) étant définies comme des zones de tissu sain (23).

2. Procédé d'analyse et de traitement d'images fluorescentes selon la revendication 1, **caractérisé en ce que** la valeur seuil est au moins approximativement égale à 30 % de l'intensité maximale (26).

3. Procédé d'analyse et de traitement d'images fluorescentes selon la revendication 2, **caractérisé en ce que** la valeur seuil est égale à 33 % de l'intensité maximale (26).

4. Procédé d'analyse et de traitement d'images fluorescentes selon la revendication 1, **caractérisé en ce que** l'intensité maximale (26) peut être déterminée automatiquement de manière autonome par le système de traitement d'image (3).

5. Procédé d'analyse et de traitement d'images fluorescentes selon la revendication 1, **caractérisé en ce que** l'intensité maximale (26) peut être déterminée manuellement par un opérateur au moyen d'un dispositif de saisie reliée au système de traitement d'image (3), dans lequel les pixels les plus clairs de l'image fluorescente (10) sont sélectionnés manuellement.

6. Procédé d'analyse et de traitement d'images fluorescentes selon la revendication 1, **caractérisé en ce que** la composante rouge (R) de l'image fluorescente (10) est convertie en image binaire par le système de traitement d'image (3) avant la détermination des lignes d'intensité limite (25).

7. Procédé d'analyse et de traitement d'images fluorescentes selon la revendication 6, **caractérisé en ce que** les lignes d'intensité limite (25) sont déterminées au moyen d'opérations de base morphologiques du traitement d'image, par exemple par dilatation et/ou érosion avec un élément structurant qui représente les limites entre le tissu tumoral 22 et le tissu sain 21.

8. Procédé d'analyse et de traitement d'images fluorescentes selon la revendication 7, **caractérisé en ce que** l'élément structurant de la dilatation et/ou de l'érosion est une matrice d'unités 3x3.

9. Procédé d'analyse et de traitement d'images fluorescentes selon la revendication 8, **caractérisé en ce que** la largeur des lignes d'intensité limite (25) est variable.

10. Procédé d'analyse et de traitement d'images fluorescentes selon la revendication 6, **caractérisé en ce que** les lignes d'intensités limite (25) sont générées au moyen d'un filtrage passe-bas ou passe-haut ou au moyen de l'application de l'opérateur de gradient.

11. Procédé d'analyse et de traitement d'images fluorescentes selon la revendication 1, **caractérisé en ce que** le système de traitement d'image (3) génère à partir de la composante rouge (R) de l'image fluorescente (10) des lignes de contour (24) fermées qui présentent une distance définie fixe par rapport à la valeur seuil définie et représentent les limites entre des surfaces d'intensité différente au-dessus et au-dessous de la valeur seuil.

12. Procédé d'analyse et de traitement d'images fluorescentes selon la revendication 1, **caractérisé en ce qu'**une image en couleurs fausses (12) est affichée par le dispositif de visualisation (4) et qui visualise par différentes couleurs les zones de tissu sain (23) et les zones de tissu tumoral (27).

13. Procédé d'analyse et de traitement d'images fluorescentes selon la revendication 12, **caractérisé en ce que** la superposition de l'image fluorescente (10) avec l'image de profil linéaire (11) et/ou l'image en couleurs fausses (12) peut être activée et désactivée.

14. Produit de programme informatique pour traiter des images fluorescentes d'un microscope opératoire médical selon l'une quelconque des revendications 1 à 13 et qui présente des parties de programme permettant d'exécuter un procédé, dans lequel le produit de programme informatique exécute les étapes suivantes consistant à :
a) extraire la composante rouge (R) de l'image fluorescente ;
b) déterminer l'intensité maximale (26) ;
c) déterminer une valeur seuil définie de l'intensité en pourcentage de l'intensité maximale (26) ;
d) générer des lignes d'intensité limite (25), qui entourent des zones de tissu tumoral (27) et dont les pixels présentent des intensités au-dessus de la valeur seuil définie, au moyen d'opérations de base morphologiques du traitement d'image, par exemple par dilatation et/ou érosion avec un élément structurant ;
e) générer une image de profil linéaire (11) qui comprend les lignes d'intensité limite générées (25) ; et
f) superposer l'image fluorescente enregistrée (10) avec l'image de profil linéaire (11), dans lequel les zones non entourées par des lignes d'intensité limite (25) sont définies comme des zones de tissu sain (23).
